# EUROPEAN PATENT APPLICATION

(11) **EP 2 957 257 A1**
(43) Date of publication of application: **23.12.2015**
(21) Application number: 15172846.6
(22) Date of filing: 19.06.2015
(51) Int. Cl.: A61F 2/04, A61F 2/962, A61F 2/24, A61F 2/00, A61B 17/00, A61B 17/068, A61B 17/064, A61F 5/00

(54) **ARTIFICIAL SPHINCTER**

(30) Priority: 20.06.2014 US 201414310403; 20.06.2014 US 201414310270; 20.06.2014 US 201414310234; 20.06.2014 US 201414310288; 20.06.2014 US 201414310313; 20.06.2014 US 201414310329; 20.06.2014 US 201414310352; 20.06.2014 US 201414310383
(71) Applicant: Ethicon Endo-Surgery, Inc., Cincinnati, OH 45242 (US)
(72) Inventor: BEDARD, Timothy S., Sewickley, PA 15143 (US); SHELTON, Frederick E., IV, Hillsboro, OH 45133 (US)
(74) Representative: Carpmaels & Ransford LLP

(57) **Abstract**

A surgical implant (900) for insertion into a biological lumen. The implant has an outer ring (915) having an outer wall and an inner wall the outer ring disposed about a central axis. The implant further has a flexible inner ring (925) adjacent to the outer ring inner wall disposed about the central axis defining an annular opening (960). The a first plurality of magnets (920) is embedded in the flexible inner ring. A second plurality of magnets (930) is located between the opening and the second plurality of magnets. The magnets are arranged around the opening to permit opening and closing of the opening through normal peristalsis.

## Description

### BACKGROUND

The present disclosure relates in general to surgical devices and procedures, and more particularly to minimally invasive surgery.

Surgical procedures are often used to treat and cure a wide range of diseases, conditions, and injuries. Surgery often requires access to internal tissue through open surgical procedures or endoscopic surgical procedures. The term "endoscopic" refers to all types of minimally invasive surgical procedures including laparoscopic, arthroscopic, natural orifice intraluminal, and natural orifice transluminal procedures. Endoscopic surgery has numerous advantages compared to traditional open surgical procedures, including reduced trauma, faster recovery, reduced risk of infection, and reduced scarring. Some forms of endoscopic surgery are often performed with an insufflatory fluid present within the body cavity, such as carbon dioxide or saline, to provide adequate space to perform the intended surgical procedures. The insufflated cavity is generally under pressure and is sometimes referred to as being in a state of pneumoperitoneum. Surgical access devices are often used to facilitate surgical manipulation of internal tissue while maintaining pneumoperitoneum. For example, trocars are often used to provide a port through which endoscopic surgical instruments are passed. Trocars generally have an instrument seal, which prevents the insufflatory fluid from escaping while an instrument is positioned in the trocar. Endoscopic surgery may also be performed in the absence of insufflatory gas. For example, minimally invasive thoracic surgery may be performed in the absence of insufflatory gas, as the rib cage provides the structure necessary to create the operating environment.

The field of gastrointestinal endoscopy has for many years focused on diagnostic and therapeutic techniques to observe, modify and remove tissues located in the digestive tract. General endoscopic procedural techniques such as visualizing, dilating, cutting and manipulating tissue have been accomplished using flexible devices such as endoscopes, balloons, snares and electrosurgical tools well known in the art.

The present disclosure concerns apparatus and methods for improving the function of biological passages. The ability of biological passages to expand and contract actively or passively to regulate the flow of solids, liquids, gases, or combinations thereof, may be compromised by defects or disease. One example of a condition associated with decreased functionality of a body passage is Gastro Esophageal Reflux Disease (hereinafter, "GERD"), which effects the esophagus. Other body passages that may be subject to dysfunction, defect, and disease include, but are not limited to, the fallopian tubes, the urethra (for example, in the case of incontinence), and the blood vessels (for example, in the case of an aneurysm). GERD and esophageal dysfunction will be further described herein for the sake of illustration.

The normal healthy esophagus is a muscular tube that carries food from the mouth through the chest cavity and into the upper part of the stomach. A small-valved opening in the distal esophagus, called the lower esophageal sphincter (hereinafter, "LES"), regulates the passage of food into the stomach. When functioning properly, the LES muscle presents a barrier to the reflux of acid or food back into the esophagus. The LES also regulates the stomach intra-gastric pressures, regulating acidic gases from refluxing from the stomach back into the esophagus. The LES, when functioning properly, will open to allow gases to be vented from the stomach. A healthy LES at rest can resist pressure from stomach gases that are at least 10 mm Hg greater than normal intragastric pressure. This pressure difference can regulate the amount of acidic fluid that refluxes from the stomach into the esophagus. The LES is controlled largely by two components.

The primary component is intrinsic smooth muscle of the distal esophagus wall. The second component is the skeletal muscle of the crural diaphragm or esophageal hiatus. The diaphragm is a muscle separating the stomach from the chest. Studies have shown that the diaphragm may act as a sphincter around the lower end of the esophagus. The esophageal hiatus is the opening in the diaphragm where the esophagus attaches to the stomach. If the LES relaxes, atrophies, or degrades for any reason, the contents of the stomach, which may be acidic, are allowed back into the esophagus resulting in reflux symptoms. The major mechanism for esophageal reflux, which may be associated with GERD, is the relaxation of one or both of the LES or hiatal diaphragm sphincter mechanisms. Normally occurring mechanisms that diminish or prevent GERD include peristaltic squeezing by the esophageal body, gravity (when a person is in an upright position), and neutralization by saliva.

Chronic or excessive acid reflux exposure may cause esophageal damage. Drugs may be required to manage symptoms of the damage and medical intervention, including surgical or endoscopic procedures, may be required to repair the damage.

The lining of the esophagus is called mucosa. Chronic exposure to stomach gases may cause the mucosa to become inflamed or ulcerated. Inflamed or ulcerated mucosa may lead to problems that may require medical intervention.

Hiatal hernias are often associated with GERD. If the esophageal hernia becomes enlarged (herniated), the LES function may be compromised and the risk of GERD increased. (A hiatal hernia occurs when the upper portion of the stomach moves up through an opening in the diaphragm.)

Barrett's Esophagus is a disease of the esophagus that may compromise esophageal function. This disease may occur when the tissue that ordinarily lines the esophagus migrates away from the lower part of the esophagus to avoid exposure to the acidic fluids against the sensitive mucosa. Barrett's Esophagus is often a precursor to esophageal cancer.

The most common symptom of GERD is dyspepsia (commonly known as "heartburn"). Dyspepsia may be defined as an acute burning sensation in the chest area typically, behind the sternum. Other symptoms of GERD may include hemorrhage, pulmonary disorders, chronic cough, intermittent wheezing, ulcers, Barrett's esophagus, and esophageal cancer.

One conventional surgical treatment for GERD is fundoplication. In this procedure, normally performed endoscopically, the upper part of the stomach is mobilized and is subsequently wrapped around the lower part of the esophagus. This highly invasive procedure is often initially successful, but has a high risk of morbidity including esophageal tears, tears of the spleen, subphrenic abcess, dysphagia, inability to vomit or belch, epigastric distension and persistent esophagitis.

Another conventional treatment for GERD is surgical suturing of a pleat of tissue between the LES and stomach to make the lower esophagus tighter. Suturing may be performed endoscopically using a suturing device on the end of an endoscope inserted into the esophagus through the mouth. Endoscopic procedures are less invasive than open surgery, but still require surgical incisions and great skill.

Surgery, whether endoscopic or open (such as fundoplication) may provide a basic mechanical correction. Surgical procedures may relocate and affix existing tissue of the stomach, esophagus, or both to add support and structure to the LES. LES strength is increased by the added support, thus reducing the incidence of reflux.

### BRIEF DESCRIPTION OF DRAWINGS

The present disclosure will be better understood from the following description taken in conjunction with the accompanying drawings illustrating some non-limiting examples. Unless otherwise indicated, the figures are not necessarily drawn to scale, but rather to illustrate the principles to be described.
Fig. 1 depicts is a sectional view of an illustrative biological passage;
Fig. 2 is an illustrative view of a human esophago-gastric junction;
Fig. 3 is an cross-sectional view of an artificial sphincter implant;
Fig. 4 is a cross-sectional view of a petal from the Fig. 3 implant;
Fig. 5 is a partial cut-away view of the Fig. 3 sphincter;
Fig. 6 is an isometric view of one example of an artificial sphincter implant;
Fig. 7A is an isometric view of an artificial sphincter implant loaded onto an endoscope;
Fig. 7B is a partial cross sectional view of the Fig. 7A implant on a deployment device;
Fig. 7C is a cross sectional view of an artificial sphincter implant deployment;
Fig. 7D is a cross sectional view of anchor deployment;
Fig. 7E is an isometric cut-away view of the Fig. 7A artificial sphincter implanted in a biological passage;
Fig. 8A is a close-up crosssectional view of implant placement before anchor deployment;
Fig. 8B is a close-up cross sectional view of anchor deployment;
Fig. 8C is a close-up cross sectional view of anchors deployed into a biological structure;
Fig. 9A is an isometric view of another example of an artificial sphincter implant;
Fig. 9B is an isometric view of another example of an artificial sphincter implant in an open state;
Fig. 9C depicts the Fig. 9B implant in a closed state;
Figs. 10A, 10B and 10C depict an artificial sphincter prior to implantation, after implantation with minor tissue in-growth and with full tissue ingrowth;
Figs. 11A, 11B and 11C depict a substance passing through one example of an artificial sphincter; and
Figs. 12A and 12B depict a substance passing through another example of an artificial sphincter.

### DETAILED DESCRIPTION

The devices and methods disclosed herein relate to providing an artificial sphincter apparatus and method for placing the same in a body lumen. The device may be used in a variety of biological lumens, including, but not limited to, the alimentary canal, particularly the distal esophagus.

The artificial sphincter may include an annular body that defines a central axis. The central axis defines an axial, or longitudinal, direction. A plane perpendicular to or nearly perpendicular to the central axis may be referred to herein as an axial plane.

At least one anchor may be coupled to the body. As used herein, anchor may refer to a single barb or quill or, anchor may refer to a plurality of barbs or quills as a collective anchor. The anchor may be configured to engage a portion of the passage, for example, a portion of a passage wall. The anchor may be configured to engage a portion of a passage inner wall. The body may include an elastic material, an inert material, a moldable material, or combinations thereof. As used herein, inert materials include materials that are biocompatible and resistant to reaction with biochemical solids, liquids, and gases. Examples include stainless steel, nickel titanium alloy, tantalum and other non-reactive metals, polymers such as PTFE, polyurethane, polyamide, and those sold under the trademark PEBAX by Elf Atochem North America, and medical grade metals and polymers.

The anchor may extend radially away from the axis and in some examples is oriented normal to an outer surface of the band portion of the artificial sphincter, or in a non-radial direction away from the axis. The anchor may extend in a curved path away from the axis. It may be pushed radially outward into the inner wall or it may be twisted or rotated about the axis to engage the inner wall.

The anchor may include an elastic material, a metal, an alloy, a polymer or combinations thereof. The anchor may also include inert material. In some examples of the artificial sphincter, the anchor may be comprised of an absorbable material (e.g. polyglycolic acid, polylactic acid, polydioxanone, PCL, organics - cellulose, collagen) and/or absorbable material that promotes tissue growth.

One or more anchors may be present in or near an axial plane of the body. The axial plane may remain stationary or nearly stationary in the longitudinal direction when the body is deformed in the radial direction. This feature can reduce stress on the passage wall near an anchor if the body deforms in connection with deployment in the passage, or if the body deforms in response to deformation of the biological passage. The biological passage may deform in response to muscular action, physiological processes, or any other processes. For example, the passage may deform in response to peristalsis, circulatory pumping, excretory processes, reproductive processes, or other physiological processes.

The anchor may be shaped like a barb or quill. In some cases, the anchor may be include a "catch". As used herein, a catch is a feature of an anchor that may be used to resist the withdrawal of the anchor from tissue. The catch may be used to pinch or pin tissue, for example, or to hook into tissue (for example, as does a barb). In some cases, the anchor may include a staple, which may be C-shaped.

The artificial sphincter may include a sheath that can be drawn over the body to protect the inner wall of a biological lumen from the anchors, for example, when the body is being inserted into the biological passage. The sheath may deflect the anchor away from a direction extending radially away from the body axis. The sheath can be removed to engage the anchor with the inner wall.

The artificial sphincter may include a coating. The coating may cover the entire surface of the body, or a portion of the body. The coating may include an inert material.

The artificial sphincter may include a therapeutic substance. For example, the substance may be present in or on the body, in the anchor, on the anchor, or both, or in or on the lining. Alternatively or additionally, the substance may be embedded in a porous portion of the artificial sphincter. The substance may elute from a portion of the artificial sphincter into the tissue of the biological passage. It may include an agent configured to heal the tissue from a disease, defect, infection, inflammation, trauma, or any combination thereof, or to physically protect the tissue from acidic compounds. For example, the substance may act to neutralize an acidic compound. The substance may be a drug, may include a steroid or may include an antibiotic.

The artificial sphincter may include anchor deployment means. These may be configured to apply force to the anchor to drive the anchor into the inner wall of the biological passage. In some of these examples, the anchor may have prongs and be shaped like a staple, a "C", a "U", a barb or any other suitable form. The deployment means may be used to drive the barbs or bend the prongs to engage the biological passage. The deployment means are used to secure the prongs to the biological passage.

Where the artificial sphincter include a moldable material, the anchor may be insert molded into a portion of the body. For example, the anchor may be wholly or partially embedded in the polymer. The anchor may be made from a material that is less elastic than the polymer. When the body is expanded away from the axis, the body portion housing the anchor may become thinner in the radial dimension. As the body portion thins, the anchor protrudes through and extends radially beyond the body portion. This feature permits the device to be inserted into the passage with the anchor in a retracted position, and permits the anchor to be exposed or "activated" automatically as the body is expanded. Where the anchor has a catch, this feature may permit the anchor to pinch a portion of the biological passage inner wall against the body when the body contracts.

The body of the artificial sphincter may include portions having different relative flexibilities, some portions of high flexibility and some portions of low flexibility. One or more anchors may be coupled to a low flexibility portion, or they may instead be coupled to a high flexibility portion. When the body expands, contracts, or otherwise deforms (for example, in response to peristalsis or other motion), the deformation is concentrated in the portions having high relative flexibility. An anchor or anchors coupled to a low flexibility portion may be subject to less stress, less displacement, or both than if coupled to a relatively high flexibility portion.

High flexibility portions may be, for example, thin, long, elastic, extensible, rotatable, or otherwise compliant. Low flexibility portions may be, for example, thick, short, inelastic, fixed against rotation or shorter, or otherwise noncompliant.

The artificial sphincter may have annular outer body with barbs protruding normally from the outer surface of the body. The annular outer body and barbs may be coated with a short term adhesive to promote fixation such as cyanoacrylate, fibrin glue or oxidized regenerated cellulose. The inner artificial sphincter body may have magnetic petals or leaves attached to or integrally formed into the inner surface of the body. The magnetic petals or leaves open as the biological lumen peristalses as will be more fully described herein. The annular body may be comprised of flexible, biocompatible material adapted for placement in a biological lumen. The artificial sphincter may be entirely over molded in a biocompatible material where that biocompatible material has nano-features to promote tissue in-growth.

The artificial sphincter may include a deployment apparatus (e.g. a catheter) for inserting the body into the biological passage.

The body of the artificial sphincter may be installed in the biological passage by placing the body in a portion of the passage, expanding the body, engaging the anchor or anchors with the passage inner wall, and allowing the body to relax. In some cases, the body is expanded and the anchor or anchors secured in the inner wall using a balloon. The body may be expanded sufficiently to partially engage the anchor or anchors with the wall. Then, a deployment means can be used to complete the engagement of the anchor or anchors with the wall. The deployment means can instead be used to secure the anchor or anchors in the wall without previous partial engagement of the anchor or anchors.

The artificial sphincter may include a coating for a portion of the biological passage, and may also include one or more anchors to secure the coating to the biological passage. The coating may include a first plurality of anchors, a second plurality of anchors, and a sleeve. The sleeve extends from the first plurality of anchors to the second plurality of anchors. The sleeve conforms to the inner wall of the biological passage. The coating may include a polymer such as PTFE, polyurethane, polyamide, and PEBAX®, any suitable medical polymer, or any suitable material that does not readily react with biological fluids such as stomach acid, biological waste, or other materials that may be present in a biological passage.

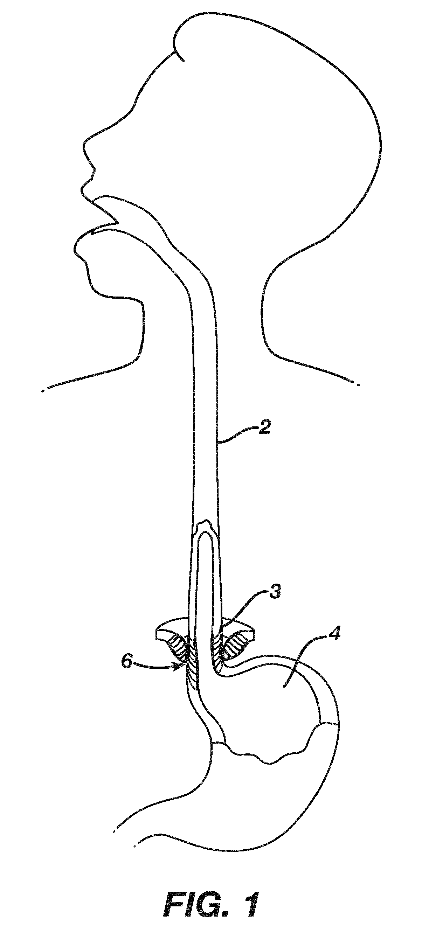

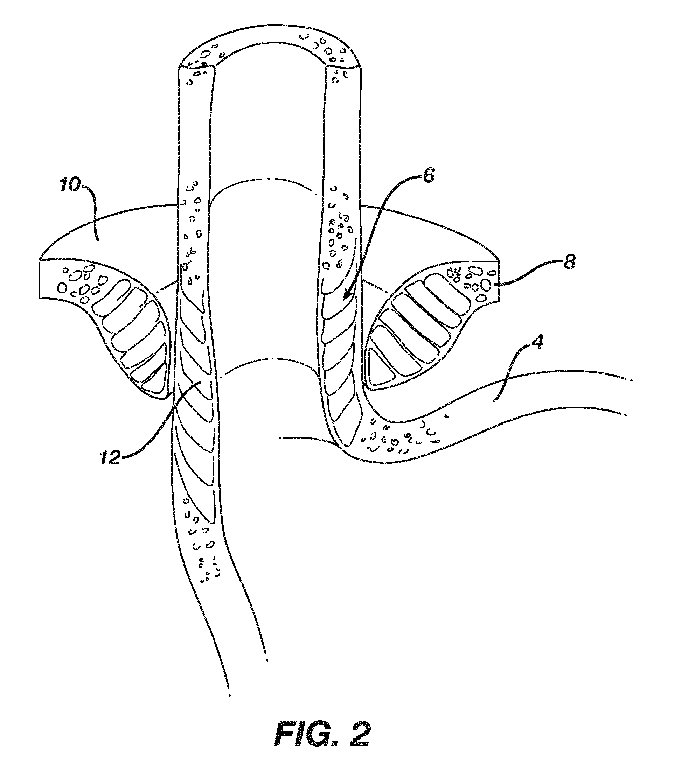

FIGS. 1 and 2 show esophageal anatomy. FIG. 1 shows a healthy esophagus 2, including the distal esophagus 3 connected to the stomach 4. The LES 6 is located at the junction of the esophagus 2 and the stomach 4 where the esophagus 2 passes through the hiatus 8 in the hiatal diaphragm 10. Sling fibers 12 in the LES 6 are smooth muscle tissue that may regulate the distal esophagus 3. The hiatus 8 may externally support and regulate the LES 6. The LES 6 is normally open at rest.

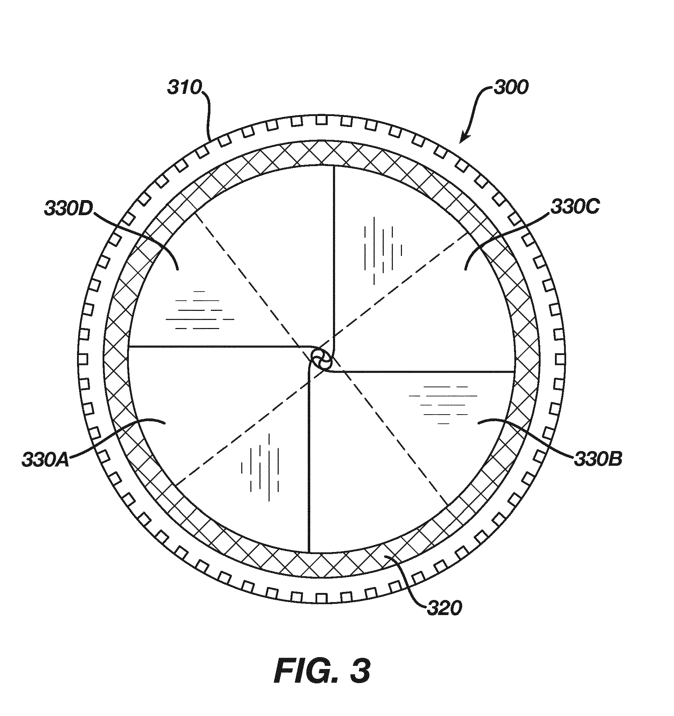

Referring now to FIG. 3, a plan view of an artificial sphincter implant 300 is depicted in a closed position. The implant 300 is annular in shape and is comprised of an outer retaining ring 310, an inner ring 320 and overlapping petals 330A-330D. Petals 330A-330D partially form an artificial valve. The rings 310 and 320 as well as the petals 330A-D may be arranged annularly around a central axis (not shown). The retaining ring 310 may be flexible in nature and may be comprised of any flexible polymer suitable for implant in a living body as is known and understood in the art. Petals 330A-330D may be comprised of a flexible bio-compatible polymer having embedded magnets with poles oriented in such a manner that the petals flex distally to permit passage of a substance e.g. food and return to the closed position. The flexible petals provide for a variably-sized bore depending upon peristaltic force as well as the size and type of substance passing therethrough.

The petals 330A-330D may be comprised of a magnetic polymer where the magnet is formed from rare earth magnets NdFeB (Neodymium Iron Boron), AINiCo (Aluminum Nickel Cobalt) SmCo (Samarium Cobalt), strontium ferrite and barium ferrite. Paramagnetism materials like: [Cr(NH3)6]Br3, K3[Cr(CN)6], K3[MoCl6], K4[V(CN)6], [Mn(NH3)6]Cl2, (NH4)2[Mn(SO4)2]•6H2O, NH4[Fe(SO4)2]•12H2O. Paramagnetism is a form of magnetism whereby certain materials are attracted by an externally applied magnetic field. In contrast with this behavior, diamagnetic materials are repelled by magnetic fields. Paramagnetic materials include most chemical elements and some compounds; they have a relative magnetic permeability greater than or equal to 1 (i.e., a positive magnetic susceptibility) and hence are attracted to magnetic fields. Ferromagnets include iron, nickel, cobalt and manganese, or their compounds (FE, CO, Ni, Gd, Dy, CrO2, MnAs, MnBi, EuO, NiO/Fe, Y3Fe5O12, and others).

To make magnets biocompatible they may be coated with or encapsulated in a non-absorbable plastic like PEEK, Polypropylene, high density polyethylene, and polycarbonate. They could also be plated or encapsulated in another non-magnetic material like Titanium. Alternatively that can be coated with a diamond-like carbon (DLC) coating or bioglass which is a commercially available family of bioactive glasses, composed of SiO2, Na2O, CaO and P2O5 in specific proportions. The proportions differ from the traditional soda-lime glasses in low amount of silica.

In order to allow elastic deformation or elastic bending of the magnet or magnets, expanded polypropylene (EPP) or high density polyethylene or an elastomer can be used e.g. isoprene or sanoprene. The coatings of DLC or bioglass can also be used for elastically deformable magnets.

The petals 330A-330D may be comprised of flexible magnets. A flexible magnet may be composed of a high-coercivity ferromagnetic compound (usually ferric oxide) mixed with a plastic binder. This is extruded as a sheet and passes on a conveyor belt over a line of powerful cylindrical permanent magnets. These magnets are arranged in a stack with alternating magnetic poles facing up (N, S, N, S,...) on a freely rotating shaft. This impresses the plastic sheet with the magnetic poles in an alternating line format. No electromagnetism is used to generate the magnets. The pole-pole distance may be on the order of 5mm, but varies with manufacturer and application. The plastics can be PEEK, Polypropylene, high density polyethylene, polycarbonate or any other biocompatible polymer. The pole-pole distance can be varied to accommodate the need to stack the petals 330 and permit them to move axially.

Unlike most conventional magnets that have distinct north and south poles, flat flexible magnets are made from composite materials and can have either a traditional thru thickness N&S two poles in one or each side or an alternating north and south poles on the same surface of the plane as is known and understood in the art.

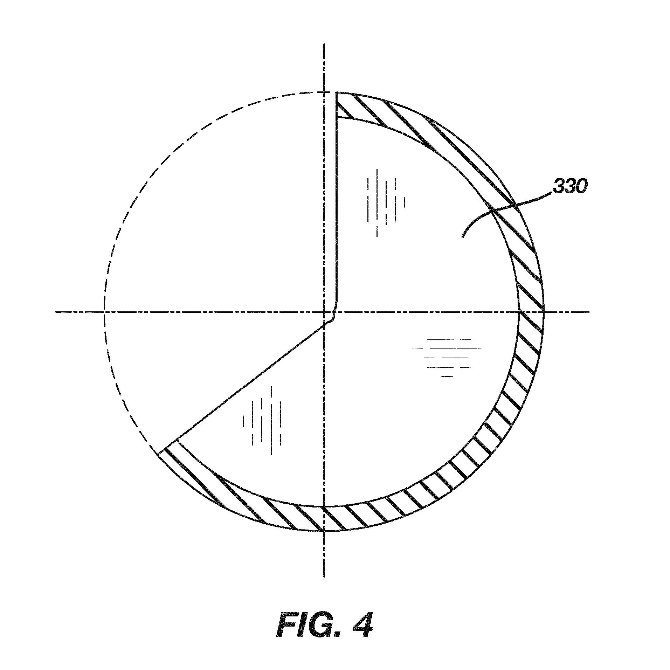

Figure 4 depicts a single petal 330C from the implant 300. As is shown in FIG. 4, the petal 300C extends approximately 225° around an axis parallel to the centerline of the ring 310 and normal to the petals 330A-330D where the axis forms the starting point of the radius of the ring 310. The remaining rings can be stacked on top of each other and spaced at 90°. The four petals, 330A-330D, overlap in a manner to completely occlude the area defined by the outer ring 310 while permitting axial movement of a substance through the implant. The petals 330, may have magnetic properties such that when interleaved or overlapped, the magnetic properties bias the artificial sphincter to a closed state.

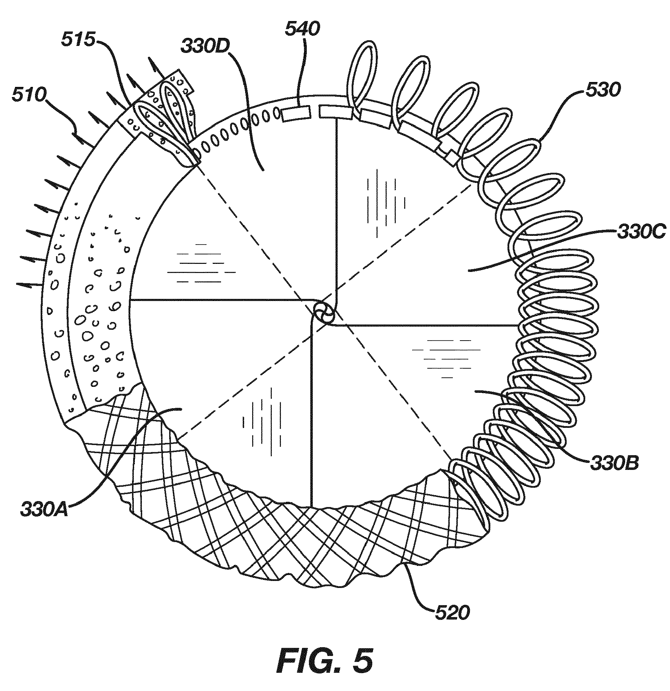

FIG. 5 depicts another example of an implant 300. The FIG. 5 implant comprises petals 330A-330D where the lateral annular edge of each petal 330 is provided with an attachment means 540 that retains a filament 530. Attachment means 540 may be a raised cut-out defining an annulus or may be hole punched in the lateral edge of the petals 330. The filament 530 is preferably flexible and is composed of bio-compatible material that maximizes flexibility and strength while minimizing the possibility of erosion through a biological structure e.g. stainless steel, nitinol, nylon, polyester, polypropylene. The filament 530 and the petals 330 may be coated or extruded with a silicone coating to minimize the possibility of erosion through a biological structure. Once the filament 530 is attached to the petals 330, an expandable, absorbable lattice structure 520 is bonded to the filament 530. The lattice structure may be formed from PGA/PCL copolymer or PLA/PCL copolymer. Those copolymers balance absorption and strength for a time period sufficient to promote tissue ingrowth while providing sufficient strength. An absorbable retainer 515 is then bonded to the lattice 520 and the filament 530.

The absorbable lattice 520 may be comprised of polyglycolic acid, polylactic acid, polydioxanone or the like. Similarly, the retainer 515 may be comprised of the same materials. The surface of the retainer 515 may be treated with biologics that encourage tissue migration and over growth. The surface of the retainer 515 may further be provided with small cleats (approximately 100-250µm tall) to promote fixation and tissue in-growth. The retainer 515 is further provided with anchors 510 oriented normal to the outer annular surface of the retainer 515. The anchors 510 penetrate the mucosa and into the muscularis of the lumen (e.g. the esophagus) to anchor the implant 300 into position during tissue in-growth. Barbs on the anchor 510 prevent the anchor 510 from inadvertently backing out of the esophageal musculari. The anchor 510 and barbs are preferably comprised of absorbable material such as PGA/PCL copolymer or PLA/PCL copolymer or any other biocompatible absorbable material having appropriate flexibility and rigidity.

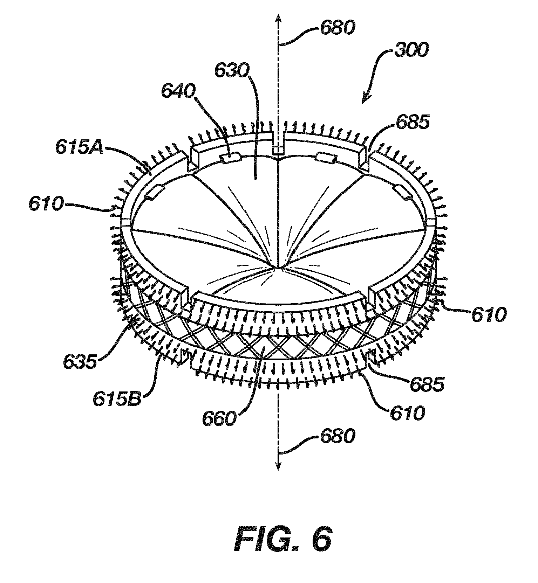

The implant 300 may have a variety of petal geometries. FIG. 6 depicts an implant 300 having a plurality of petals 630 that do not overlap. As shown, the implant 300 is comprised of an outer retaining ring 615A. Like the FIG. 5 example, these retaining rings 615 may be absorbable. The ring 615 is configured with a non-absorbable band 660 that serves to retain and orient a plurality of petals 630. The ring 615 is fixedly attached to the band 660 and may be comprised of two rings 615A and 615B oriented above and below the band 660 along a central axis 680. Alternatively, the rings 615A and B and the ring 660 may be one contiguous structure. The petals 630 may each be configured with internal magnets to fix the petals 630 in the closed position as shown in FIG. 6. The petals 630 may be a polymer having magnets disposed therein or may be a ferric oxide mixed with a polymer or plastic to impart magnetic properties to the petals 630. The magnetic properties and orientation of the poles of the petals 630 are such that the petals 630 are biased to remain in contact at a resting state. When all the petals 630 are in contact, the implant is in a closed state. The FIG. 6 implant is further provided with anchors 610 that serve to fix the implant 300 in a hollow biological structure. As shown, the anchors 610 are comprised of quills oriented normal to the exterior surface of the rings 615A and 615B. The rings 615A & B are configured with notches 685 to promote flexibility and permit the rings 615A & B to be folded medially toward the axis 680.

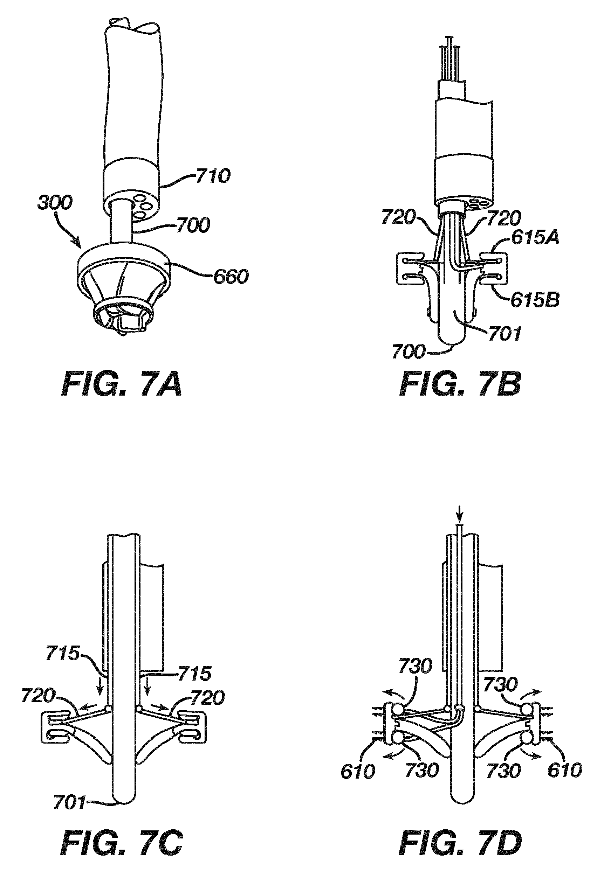

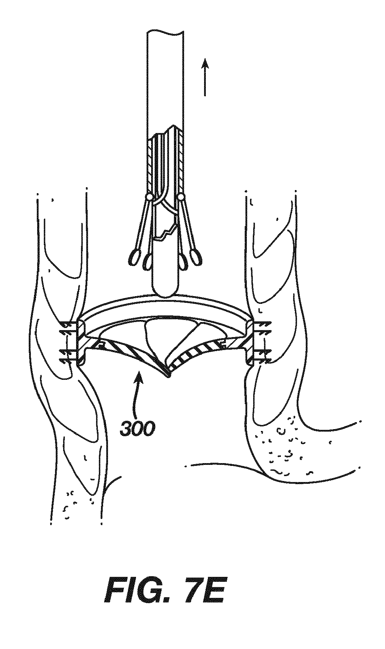

Referring now to FIGS. 7A - 7E, an implantation process utilizing the implant 300 is shown. In FIG. 7A, the implant 300 is oriented about a deployment mechanism 700 in a retained state. As is shown in FIG. 7A, the deployment mechanism is extending from a working channel of an endoscope 710. The deployment mechanism 700 is provided with an elongate shaft 705 of suitable length to fit down an endoscope working channel. A sheath 715 is disposed about the shaft 701. At the distal end of the shaft 701, a plurality of arms 720 are flexibly attached to the sheath 715 and arranged annularly about the shaft 701. In the FIG. 7 implant, four arms 720 are provided but it is contemplated that as few as two and as many as 12 or more arms 720 may be provided. It is further contemplated that the arms 720 may be staggered such that alternating arms interact with the upper 615A and lower 615B portions of the ring 660.

As is shown, the rings 615A and 615B are folded medially. The interior surface of the rings 615A and 615B may be provided with annuli that mate with protrusions on the deployment arms 720, FIG. 7B. When the deployment arms 720 are extended as in FIG. 7C, the implant 300 expands to contact the interior lumen walls of an anatomic structure e.g. the esophageal mucosa. Upon contact with the interior wall, a second deployment apparatus 730 is utilized to move the rings 615A and 615B into contact with the interior wall. As depicted, small balloons 730 are utilized to move the rings 615A and 615B into contact thereby deploying the anchors 610 into the lumen interior walls. Once the anchors 610 are deployed, the deployment mechanism 700 is removed as is shown in FIG. 7E. The distal arms used to deploy the ring 615B are pulled through the petals 630. Due to the magnetic orientation of the petals 630, the petals are biased back to a closed state as shown in FIG. 7E after removal of the deployment mechanism 700. Although FIGS. 7A - 7E depict deployment of the FIG. 6 implant 300, it is understood that the deployment mechanism 700 may be used with the FIG. 3 implant as well.

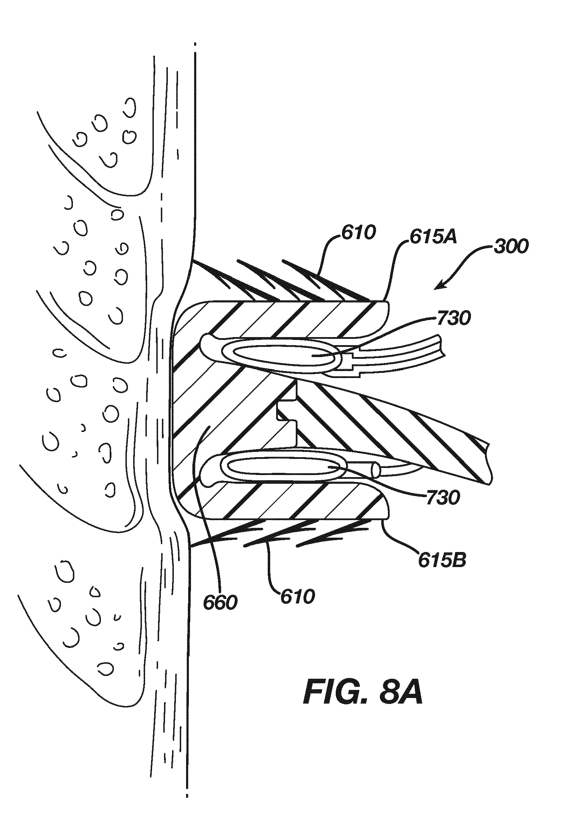

Referring now to FIG. 8A, a close up view of the rings 615A and 615B as well as the anchors 610 is shown. In this view, the rings 615A and 615B are shown folded medially about the balloon 730. As stated above, the medial surface of the rings 615A and 615B may be provided with annuli (not shown) that mate with projections (not shown) on the balloons 730 to retain the rings 615A and 615B in the undeployed state. Alternatively, the balloons 730 may be provided with a pressure-sensitive adhesive that retains the rings 615A and 615B in the undeployed state. As shown, the anchors 610 are disposed about the rings 615 in less than a normal orientation and may further rest against the outer surfaces of the rings 615 until the rings 615 are deployed. This anchor 610 orientation aids in insertion into a biological lumen, e.g. the esophagus. As shown in FIGS. 8A-8C, the rings 615A and 615B and the ring 660 are of unitary construction. As discussed above, the rings 615 and 660 may be discrete components or a unitary ring as shown.

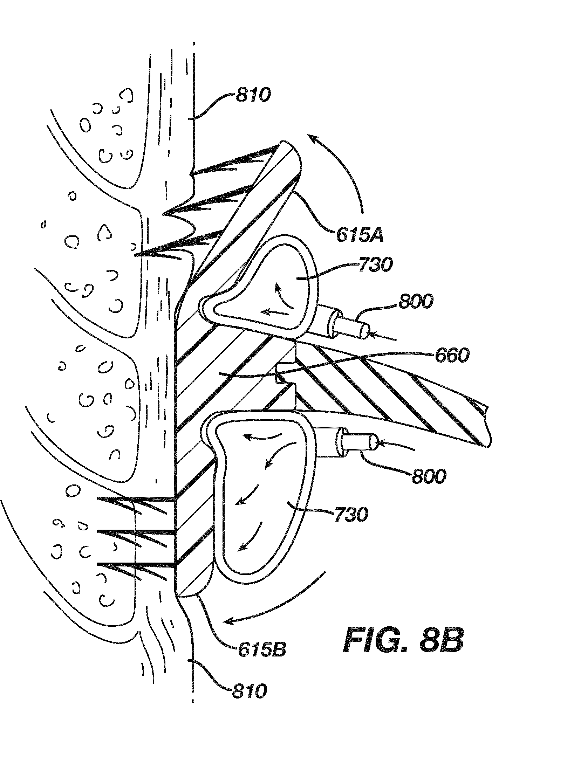

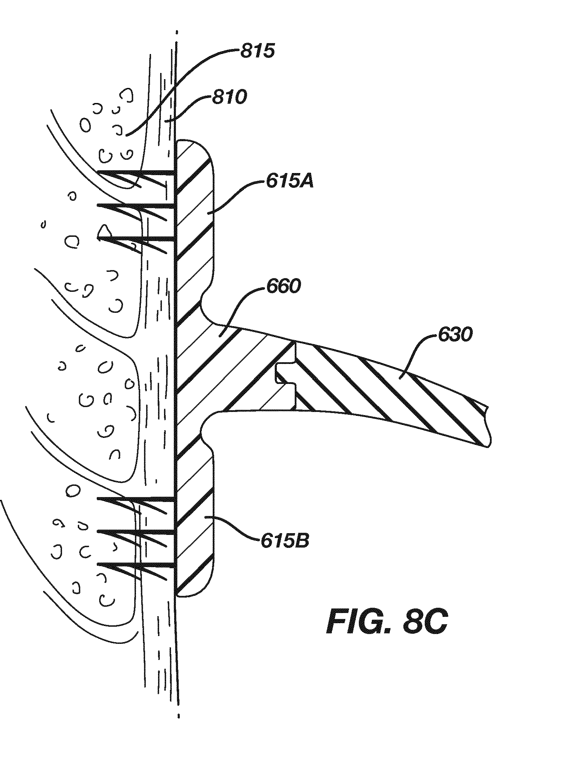

Once the implant 300 is located in an appropriate deployment position in a biological lumen, the arms 720 are deployed. Once the arms 720 are deployed, the implant 300 is biased against the lumen wall. The balloons 730 are then inflated as shown in FIG. 8B. As the balloons 730 are filled with an appropriate substance, e.g. saline via fill tubes 800, the rings 615 move from a medial folded position to contact the lumen wall 800. As the rings 615 deploy, the anchors 610 or quills 510 pierce the lumen wall sufficiently to retain the implant 300 in a fixed position relative to the lumen wall 810 as shown in FIG. 8C. As shown in FIG. 8C, the quills or anchors 510, 610 may penetrate through the submucosa and into the muscularis 815.

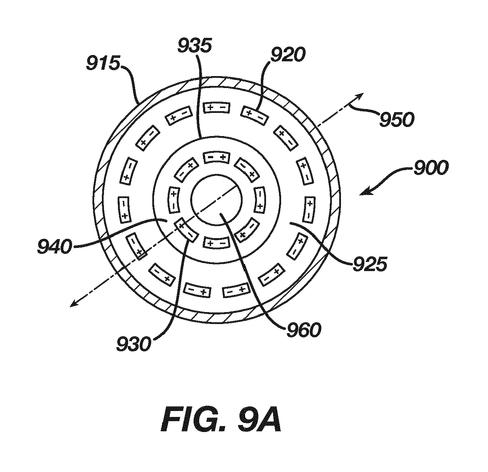

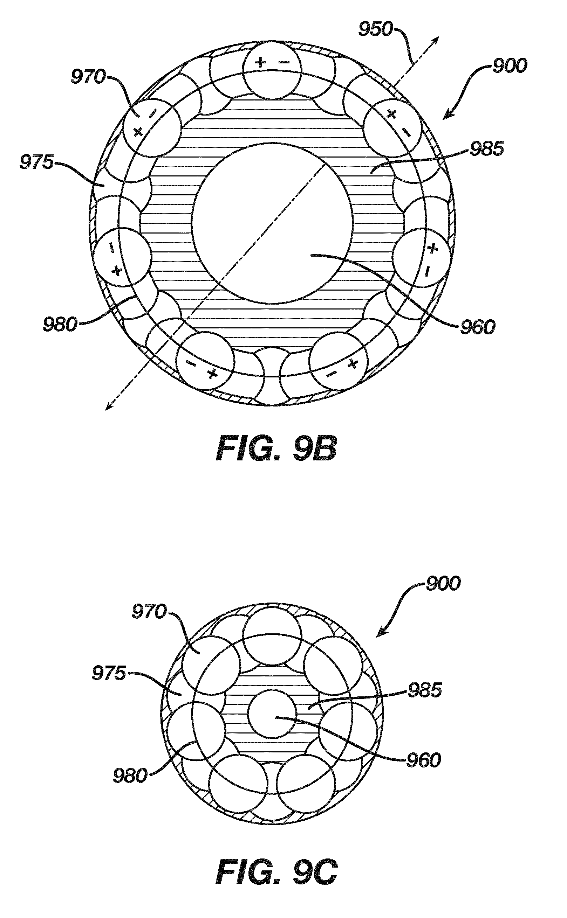

Referring now to FIGS. 9A - 9C, another example of an implant 300 is depicted as implant 900. The FIG. 9 implant has an outer ring 915 that may have outer rings as set forth in FIGS. 5 and 6 along with anchors or quills disposed about the outer surface of the ring 915. In the FIG. 9 implant, a plurality of magnets 920 is oriented such that their poles are arranged end to end in an approximately annular arrangement about the axis 950. As shown, the magnets 920 are encased in a highly flexible silicone sheath 925 which permits the magnets to move toward and away from each other thereby pursing the sheath 925. Another plurality of magnets 930 may be arranged in the same manner as the magnets 920 and enclosed in highly flexible silicone sheath 940 where the sheath permits the movement of the magnets 930 toward each other but prevents the magnets 930 from overlapping with the first magnets 920. This arrangement of magnets 920 and 930 allows silicone 925 and 940 to bunch or purse thereby occluding a lumen forming a valve. However, the magnetic force between the magnets 920 and the force between the magnets 930 is not so great that normal peristalsis cannot force a substance, e.g. food or water through the opening 960. As shown, the opening 960 is in a partially opened state. In a resting state, silicone 940 would be occluded thereby preventing passage of food or water or any other substance.

As shown in FIG. 9A, the magnets 920 and 930 are arranged in an annular fashion about the axis 950 where the axis 950 defines a central lumen through the implant 900. The magnets 920 and 930 may be organized in any fashion to accomplish the goal of permitting opening and closing of the opening 960 through normal peristalsis. In the FIG. 9A example, a plurality of outer ring magnets 920 are embedded in a flexible biocompatible material e.g. silicone forming an outer magnetic ring. A plurality of inner ring magnets 930 are embedded in flexible biocompatible material as well. The polarities of the rings 920 and 930 are arranged in such a fashion that the ring 920 repels the ring 930 thereby biasing the implant to a closed state. The magnets of the ring 930 are oriented with respect to each other such that they occlude the opening 960. The relative repulsion between the rings 920 and 930 and the magnetic strength of the magnets of the ring 930 are such that normal peristalsis forces individual magnets in the rings 920 and 930 to move away from each other such that the opening 960 is created permitting passage of food.

Referring now to FIG. 9B, another implant 900 is shown. This example possesses magnets 970 that are generally spherical and arranged about the axis 950. The magnets 970 are spaced apart by spacers 975 and the magnets 970 are retained relative to one another by a cord 980 where the cord 980 passes through annuli in the magnets 970 and the spacers 975. The cord 980, magnets 970 and spacers 975 are provided in a highly flexible silicone bag 980 such that when the magnets 970 and spacers 975 are in a relaxed state, the bag 980 occludes the opening 960 (see FIG. 9C). As in previous examples, the FIG. 9B implant may be provided with rings 915 and anchors or quills as set forth above.

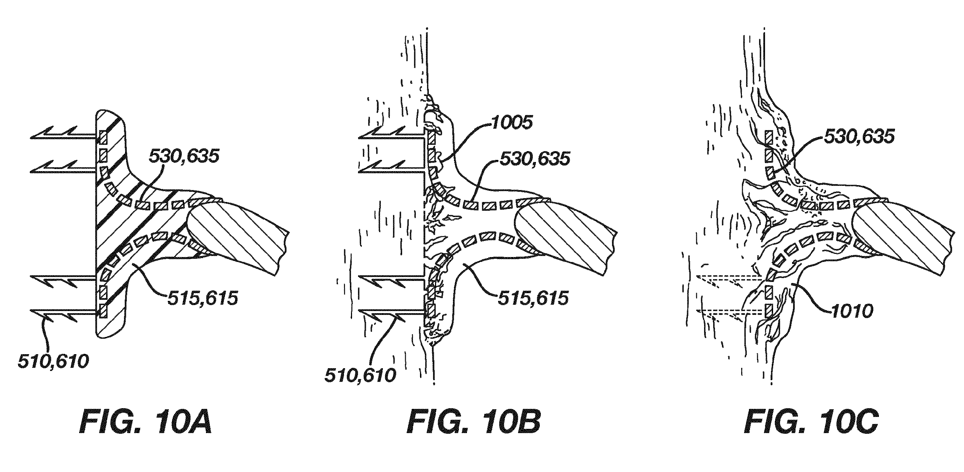

Referring now to FIGS. 10A - 10B, the implant 300 is shown at various times after implantation. FIG. 10A depicts the implant 300 just after implantation where implant fixation is reliant upon the holding strength of anchors or quills 510, 610. FIG. 10B depicts the ring 515, 615 beginning to break down thereby permitting and stimulating tissue in-growth 1005. The ring 515, 615 may be comprised of materials that promote tissue growth and, for example, the ring 515, 615 can include both hydrophilic portions and hydrophobic portions to form a hydrophilic-hydrophobic adjunct material. The resulting combination can advantageously have surfaces or portions that attract cells and encourage cell ingrowth (hydrophilic) and surfaces or portions that do not attract cells or otherwise encourage cell ingrowth (hydrophobic). The hydrophobic portions can be placed in contact with the tissue, while the hydrophobic portions can be oriented away from the tissue surface.

Synthetic polymers can be used to form adjunct materials that are hydrophobic, such as polycaprolactone (PCL) and polylactic acid (PLLA). It is noted that "polymers" as used herein can include copolymers. Synthetic adjunct materials, however, can be treated or otherwise produced to be hydrophilic, as will be discussed herein. To form the adjunct material, any method of creating a synthetic material having a hydrophilic portion and a hydrophobic portion can be used. In some cases, a surface of (or only half of) a hydrophobic adjunct material is treated with an acid or base which can cause the formation of pockets or pits in the surface. Alternatively, the adjunct material can be formed by bonding a hydrophilic layer to a hydrophobic layer. For example, an adjunct material can be treated such that the entire adjunct material becomes hydrophilic. Then this hydrophilic layer can be bound, such as by laminating, to a second hydrophobic adjunct material layer creating a material that is hydrophobic and hydrophilic. Various approaches can be used to create an adjunct material or matrix where a tissue contacting portion encourages cellular ingrowth while a non-tissue contacting portion discourages cellular ingrowth.

Referring now to FIG. 10C, the anchors 510, 610 and the rings 515, 615 have been fully absorbed. Mucosa 1010 is fully grown into the scaffold 530, 635.

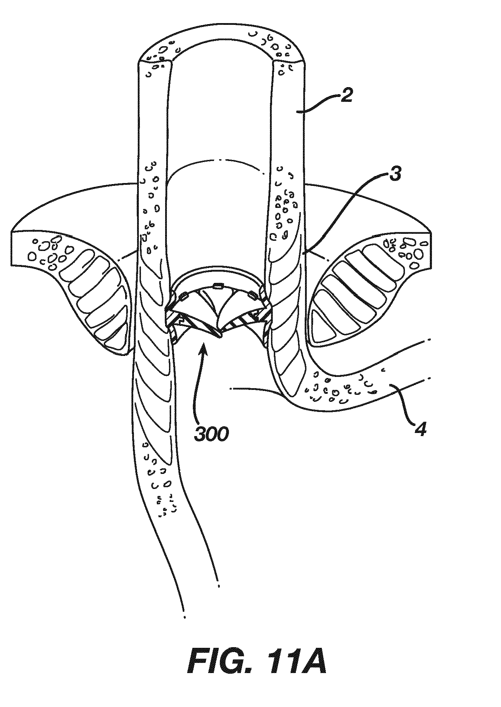

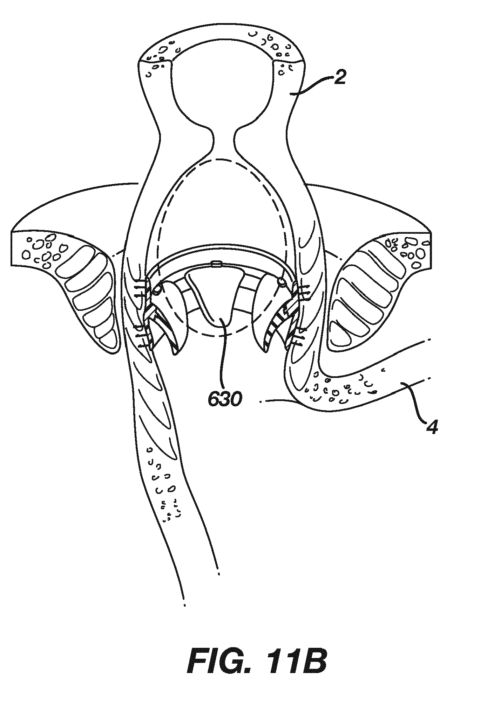

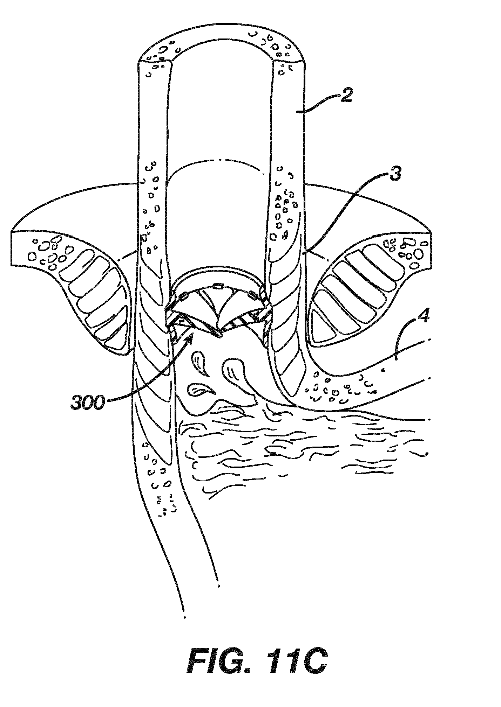

After implanting, the implant 300, as depicted in FIG. 11A, is situated in the esophagus 2 and is substantially located at the junction of the esophagus 2 and the stomach 4. Referring now to FIG. 11B, the implant 300 and the petals 630 are depicted in an "open" state in response to esophageal peristalsis. A bolus of food is shown passing through the opening created by the expansion of the implant 300 as well as the distal extension of the petals 630. After the food passes through the implant 300, the petals 630 return to a closed state as shown in FIG. 11C.

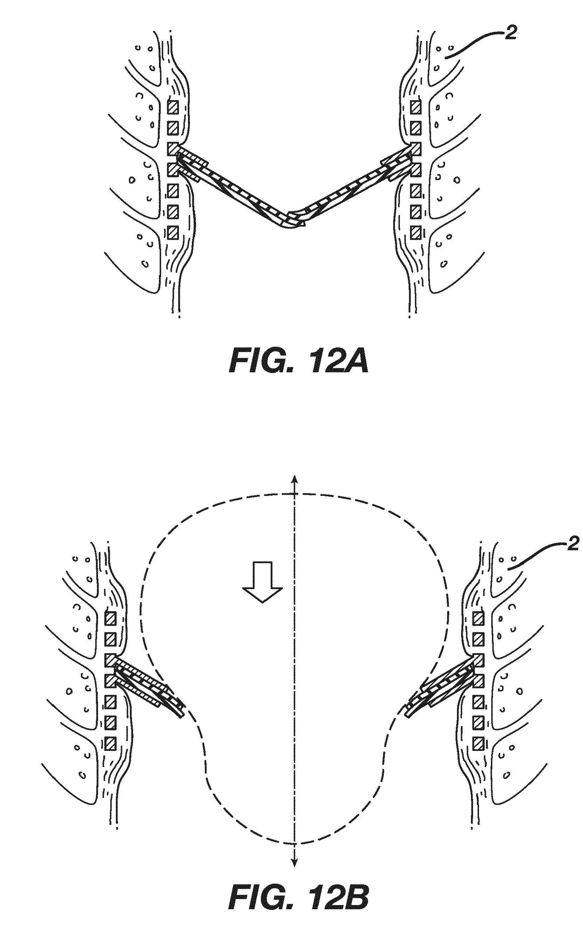

Referring now to FIG. 12A, a cross-section of the FIG. 5 implant 300 is shown in an implanted state. The overlapping nature of the petals 330 is depicted. As the lumen 2 expands through a peristaltic wave, the implant 300 expands normal to the axis 1200 (the direction of peristalsis and food movement) as shown in FIG. 12B. This normal expansion creates an opening about the axis 1200 in the petals 330, permitting passage of food past the implant 300.

## Claims

1. A surgical implant, comprising:
an outer ring having an outer wall and an inner wall, the outer ring disposed about a central axis;
a flexible ring adjacent the inner wall, the ring extending annularly from the inner wall towards the central axis, the flexible ring having an opening disposed about the central axis;
a first plurality of magnets embedded in the flexible ring, the first plurality of magnets arranged in an annular fashion about the central axis; and
a second plurality of magnets disposed about the central axis, the second plurality of magnets located between the opening and the first plurality of magnets.

2. The surgical implant of Claim 1, further comprising a plurality of anchors disposed about the outer ring outer wall.

3. The surgical implant of Claim 2 wherein the plurality of anchors are oriented substantially perpendicularly to the outer ring outer wall.

4. The surgical implant of Claim 2 or Claim 3, wherein the anchors have a barb to promote fixation in tissue.

5. The surgical implant of any one of Claims 2 - 4, wherein the outer ring and the anchors are comprised of a bio-absorbable material.

6. The surgical implant of Claim 1, wherein:
the outer wall and inner wall of the outer ring define a ring thickness, the top and bottom of the outer ring defining a height;
a plurality of notches extend from the outer wall towards the central axis;
the flexible ring is located in the middle of the outer ring height, the flexible ring extending annularly from the inner wall towards the central axis.

7. The surgical implant of Claim 6 wherein the outer ring and the inner ring are of unitary construction.

8. The surgical implant of any preceding Claim wherein the inner ring and the outer ring are bio-absorbable.

9. The surgical implant of Claim 8 further comprising a lattice structure embedded within the outer ring.

10. The surgical implant of Claim 9 wherein the lattice structure is bio-absorbable.

11. The surgical implant of Claim 10 wherein the inner ring and the outer ring are bio-absorbable and wherein the outer ring and inner ring absorb faster than the lattice structure.

12. The surgical implant of any one of Claims 6 - 11 further comprising a plurality of anchors disposed about the outer ring outer wall.

13. The surgical implant of Claim 17 wherein the anchors are bio-absorbable.

14. The surgical implant of Claim 12 or Claim 13 wherein at least two of the anchors have barbs.

15. A surgical implant, comprising:
an outer ring having an outer wall and an inner wall, the outer ring disposed about a central axis;
a first flexible ring adjacent the inner wall, the first flexible ring extending annularly from the inner wall towards the central axis;
a first plurality of magnets embedded in the first flexible ring, the first plurality of magnets arranged in an annular fashion about the central axis;
a second flexible ring located between the first flexible ring and the central axis, the second flexible ring having an opening disposed about the central axis; and
a second plurality of magnets disposed about the central axis, the second plurality of magnets located substantially within the second flexible ring.

16. The surgical implant of Claim 15 wherein the first and second pluralities of magnets bias the opening to a first state.

17. The surgical implant of Claim 15 or Claim 16 wherein the first plurality of magnets and second plurality of magnets are arranged such that the first flexible ring repulses the second flexible ring.

18. The surgical implant of any one of Claims 15 - 17, wherein the first flexible ring is comprised of silicone.

19. The surgical implant of any one of Claims 15 - 18, wherein the second flexible ring is comprised of silicone.
